Europäisches Patentamt

European Patent Office

Office européen des brevets

(11) Numéro de publication: **0 402 186**
**A2**

## (12) DEMANDE DE BREVET EUROPEEN

(21) Numéro de dépôt: **90401271.3**

(22) Date de dépôt: **15.05.90**

(51) Int. Cl.⁵: **C08B 30/06, A61K 9/20, C08J 3/12, //C08L3:02**

(30) Priorité: **16.05.89 FR 8906385**

(43) Date de publication de la demande:
**12.12.90 Bulletin 90/50**

(84) Etats contractants désignés:
**AT BE CH DE DK ES FR GB GR IT LI LU NL SE**

(71) Demandeur: **S.E.P.P.I.C., SOCIETE D'EXPLOITATION DE PRODUITS POUR LES INDUSTRIES CHIMIQUES**
**75, quai d'Orsay**
**F-75321 Paris Cédex 07(FR)**

(72) Inventeur: **Brancq, Bernard**
**2, rue d'Armenonville**
**F-78150 Le Chesnay(FR)**
Inventeur: **Trouve, Gérard**
**134 Chemin du Rozé**
**F-81100 Castres(FR)**

(74) Mandataire: **Portal, Gérard et al**
**Cabinet Beau de Loménie 55, rue d'Amsterdam**
**F-75008 Paris(FR)**

(54) Procédé de fabrication d'un amidon directement compressible en vue de l'utilisation dans la fabrication de comprimés et comprimés obtenus.

(57) La présente invention concerne un procédé de préparation de grains d'amidon, caractérisé en ce que l'on réalise la granulation d'un mélange obtenu par pulvérisation de 1 à 20 %, et de préférence de 2 à 6 % en poids, d'un empois d'amidon obtenu, à partir d'amidon natif, dans des conditions douces et de 99 à 80 % en poids de grains d'amidon natif et que le produit obtenu est séché, broyé et tamisé de façon à obtenir des agrégats (grains) ayant une granulométrie moyenne comprise entre environ 100 et environ 500 μm.

EP 0 402 186 A2

## Procédé de fabrication d'un amidon directement compressible en vue de l'utilisation dans la fabrication de comprimés et comprimés obtenus.

Parmi les formes galéniques sous lesquelles les principes actifs thérapeutiques sont administrés, le comprimé tient une place particulièrement importante. Ces comprimés sont réalisés par compression de mélanges contenant, outre le ou les principes actifs, un ou plusieurs excipients permettant l'obtention de comprimés ayant les propriétés recherchées. Parmi ces excipients, l'un des plus couramment utilisés est l'amidon.

L'amidon natif (qui peut être d'origines très diverses) se présente sous la forme de grains dont le diamètre moyen est de quelques dizaines de microns (généralement 5 à 40 $\mu$m) ; cet amidon natif s'écoule mal, est très difficile à comprimer et ne donne pas naissance à des comprimés ayant une dureté suffisante.

On a proposé de modifier cet amidon natif afin d'en améliorer les propriétés ; les modifications décrites sont de deux types :

- les unes consistent à réaliser des modifications chimiques de l'amidon ou de la surface des grains d'amidon, ce qui conduit à des produits ne répondant plus à la monographie "amidon" des pharmacopées. Un exemple de ce type de produit est décrit dans le brevet EP 159 631 ;

- les autres consistent à réaliser des modifications physiques tendant à gélatiniser partiellement la surface des grains d'amidon en les faisant passer sur des rouleaux chauffés ou en les soumettant à des pressions élevées en présence d'eau. Des modifications de ce type sont décrites dans les brevets US 3 622 677 et US 4 072 535 ; ces brevets décrivent le procédé de fabrication et l'utilisation de l'amidon pris comme élément de comparaison dans les exemples de la présente demande de brevet.

Les produits obtenus par modification physique appartiennent à la classe des "amidons prégélatinisés" ; ils présentent souvent un écoulement insuffisant, un mauvais pouvoir désintégrant mais une comprimabilité améliorée.

On a également préconisé de réaliser des pâtes en mélangeant des grains d'amidon natif avec de l'empois d'amidon de viscosité relativement élevée et en forçant ce mélange à travers les mailles d'un tamis. Mais les résultats fournis montrent que l'on n'obtient pas, par mise en oeuvre de cette méthode, d'amélioration substantielle des propriétés de l'amidon natif par rapport aux amidons commerciaux connus, que la proportion d'empois soit faible (cf Pharm. WEEKBLATT, 9, (5) 1987 p 274) ou élevée (cf brevet US 3 171 747).

La présente invention vise à l'obtention de granulés d'amidon directement compressible présentant, par rapport aux amidons décrits, une granulométrie adéquate pour un mélange homogène avec des principes actifs, des propriétés nettement améliorées pour ce qui concerne notamment la granulométrie, l'écoulement, l'aptitude à la compression et la rapidité de désagrégation des comprimés obtenus.

Le procédé selon l'invention est caractérisé en ce qu'on réalise la granulation d'un mélange obtenu par pulvérisation de 1 à 20 % et de préférence de 2 à 6 % en poids d'un empois d'amidon obtenu, à partir d'amidon natif, dans des conditions douces, et sur 99 à 80 % en poids de grains d'amidon natif, que le produit obtenu est séché puis éventuellement broyé et tamisé de façon à obtenir des agrégats de grains d'amidon, appelés granulés, ayant une granulométrie moyenne comprise entre environ 100 et environ 500 $\mu$m.

Dans le procédé selon l'invention, l'"empois d'amidon" est obtenu, à partir d'amidon, dans des "conditions douces". On sait que l'empois d'amidon est très généralement obtenu par chauffage d'une dispersion aqueuse d'amidon natif. Selon la vitesse de chauffage, selon la température atteinte, selon la durée dudit chauffage, l'amidon est plus ou moins dégradé ; on vise selon l'invention à la mise en oeuvre de conditions douces, c'est-à-dire d'un chauffage progressif, d'une température maximum ne dépassant pas environ 85° C et d'une durée de l'ordre de 15 à 45 min.

L'empois d'amidon ainsi obtenu se présente sous la forme d'une solution aqueuse dont la concentration (en amidon dans l'empois) est de l'ordre de 2 à 10 %, de préférence de 5 à 8 % en poids et dont la viscosité est inférieure à 1 500 mPa.s à 85° C, pour une concentration de 5 % en amidon. Ces solutions aqueuses sont ainsi suffisamment fluides pour pouvoir être aisément pulvérisées.

On a pu mettre en évidence deux caractéristiques complémentaires de cet empois d'amidon permettant l'obtention de produits finaux présentant des propriétés optimales :

- d'une part, il est souhaitable que l'empois d'amidon soit utilisé aussitôt que possible après sa préparation ; on emploiera donc, pour la pulvérisation, la solution chaude (température au moins égale à 60° C) obtenue à la fin du procédé de préparation de l'empois,

- d'autre part, il est souhaitable que l'empois d'amidon soit obtenu à partir du même amidon natif (tout au moins d'un amidon de même origine) que celui sur lequel ledit empois sera granulé.

La granulation est réalisée dans un appareil de type mélangeur granulateur (DIOSNA, LODIGE, GUEDU, FIELDER) ou dans tout autre appareil ayant les mêmes caractéristiques.

L'agitateur de l'appareil est mis en route puis l'empois d'amidon chaud est pompé et pulvérisé sur les grains d'amidon en mouvement. L'agitation est maintenue après introduction de tout l'empois pendant un temps suffisant pour obtenir une masse humide granulée homogène, non collante, ne présentant pas d'agglomérats non friables de grosse taille. La mise en route du dispositif de démottage, disponible sur certains des appareils mentionnés, est souhaitable pendant la dernière phase de la granulation.

Les granulés obtenus dans l'opération de granulation sont séchés dans un appareil de séchage conventionnel qui peut être une étuve ventilée, une étuve à vide ou un lit d'air fluidisé. La température de l'air ambiant dans le dispositif de séchage ne devra pas dépasser 90 °C de préférence.

Une variante du procédé consiste à réaliser la granulation et le séchage en une seule étape dans un lit d'air fluidisé équipé d'un dispositif de pulvérisation, ou dans un granulateur-sécheur, du type par exemple de celui proposé par la société MORRITZ.

Les granulés secs obtenus sont ensuite éventuellement broyés dans un broyeur classique (par exemple à marteaux, à couteaux ou à billes) ou par action d'une râpe ; ce broyage doit être conduit de façon telle que l'on obtienne, après tamisage, des granulés présentant une granulométrie moyenne de 100 à 500 µm environ.

Les granulés secs obtenus par mise en oeuvre du procédé selon l'invention présentent une structure particulière que l'on peut décrire schématiquement comme suit ; lesdits granulés sont formés par le collage superficiel et ponctuel, par le moyen de l'empois d'amidon, de plusieurs (par exemple de 100 à 1 000) grains d'amidon natif, ces grains d'amidon natif ne présentant pas en eux-mêmes une modification notable de la structure de leur surface.

Ces granulés qui ont une granulométrie moyenne comprise entre environ 100 et 500 µm donnent, par compression sous 20 daN, des comprimés ayant une dureté d'au moins 50 N environ et se désintégrant dans l'eau en moins de 5 min.

Le procédé décrit dans la présente invention permet l'obtention d'un amidon directement compressible correspondant à la monographie "amidon prégélatinisé" des différentes pharmacopées. Cet amidon, comparativement aux produits existant sur le marché se caractérise par :
- un très bon écoulement assurant une excellente régularité de poids des comprimés,
- une bonne aptitude à la compression,
- un pouvoir désintégrant particulièrement élevé.

L'amidon obtenu est bien entendu utilisable pour les applications connues de l'amidon natif et notamment pour la préparation de comprimés ; dans cette utilisation, ledit amidon est utilisé seul ou avec les autres excipients (et additifs) connus et utilisés.

Les exemples non limitatifs suivants illustrent l'invention.


EXEMPLE 1


Fabrication d'un lot pilote d'amidon compressible


- Empois d'amidon

On verse 567 g d'amidon de maïs CODEX (Fleurine 3401 -CERESTAR) dans 10,31 l d'eau froide sous agitation (agitateur Raynerie, muni d'une défloculeuse). La dispersion est chauffée progressivement, pendant 1 h jusqu'à ce que la température atteigne 85 °C, puis est maintenue à cette température pendant 30 min. Un ajustement en eau est effectué pour compenser l'évaporation. La solution à 5 % obtenue présente, à 85 °C, une viscosité de l'ordre de 770 mPa.s.


- Granulation

20 kg de Fleurine 3401 sont déposés dans un granulateur DIOSNA modèle V 100 L'agitation du granulateur est mise en route (vitesse 1) et l'empois est pompé au moyen d'une pompe péristaltique en 2,5 min. L'agitation est maintenue en l'état pendant 10 min au total puis le démotteur est mis en marche pendant 4 min supplémentaires (vitesse I).

Le grain obtenu est séché dans une étuve ventilée MEMMERT de 240 l à 80° C jusqu'à une humidité résiduelle de 12 %.

Il est ensuite broyé dans un broyeur TORNADO (Sharples, Stokes, Rueil Malmaison) muni d'une grille fine de 355 μm.

- Caractéristiques du granulé obtenu

Elles sont résumées dans le tableau 1. Les méthodes de mesures sont décrites dans les références ci-dessous :
- Guyot J.C., Delacourte A., Marle B.
Drug Dev. Ind. Pharm. 12 1869-1884 (1986)
- Guyot J.C.
S.T. Pharma. 7, 10, 551-559 (1978)
- Delacourte A., Guyot J.C., Traisnel M
S.T. Pharma. 11, 3, 131-140 (1982)

- Formulations

Deux formules contenant des principes actifs ont été étudiées :

| FORMULE 1 : | Aspirine cristallisée | 80 % |
| | Amidon | 20 % |
| FORMULE 2 : | Acide ascorbique | 62 % |
| | Amidon | 32,5 % |
| | Stérotex (R) | 2 % |
| | Aérosil (R) | 0,5 % |

Les caractéristiques des comprimés obtenus sont données dans le tableau 2.

## EXEMPLE 2

On fabrique 3,5 kg d'un empois d'amidon à 5,5 % d'amidon selon le procédé décrit dans l'exemple 1.

Cet empois est versé en 1 min dans le granulateur DIOSNA contenant 7 kg de Fleurine 3401. La granulation est effectuée selon la description de l'exemple 1.

Le grain obtenu est séché dans un lit d'air fluidisé à 80° C (Aeromatic STREA 1) jusqu'à une humidité finale de 12 % puis broyé comme il est dit à l'exemple 1.

Les caractéristiques du grain sont données dans le tableau 1.

## EXEMPLE 3

On fabrique 78 kg d'un empois d'amidon à 5,5 % en chauffant lentement pendant 2 h une dispersion de Fleurine 3401 dans de l'eau jusqu'à ce que la température de 85° C soit atteinte. Cette température est maintenue pendant 30 min puis l'empois est pompé en 11 min dans un granulateur DIOSNA de 600 l contenant 150 kg de Fleurine 3401.

L'agitation du DIOSNA (vitesse 1) est maintenue au total 21 min au bout desquelles on effectue encore 4 min de démottage à vitesse 1.

Le grain obtenu est séché dans une étuve à vide à 80° C sous environ 5 000 Pa pendant 14 h jusqu'à ce qu'une humidité résiduelle de 11 % soit atteinte. Il est ensuite broyé comme précédemment décrit.

Les caractéristiques des grains obtenus et des formules réalisées sont données dans les tableaux 1 et 2.

## EXEMPLE 4

Réalisation d'un grain par granulation-séchage dans un lit d'air fluidisé

500 g d'amidon de maïs CODEX sont placés dans un lit d'air Aeromatic STREA 1. Le débit d'air de sustentation est réglé à 120 m³/h. La température d'entrée de l'air est de 20° C, la température de sortie est 80° C.

600 g d'un empois d'amidon à 5 % sont pulvérisés sur les grains d'amidon au moyen d'une buse de pulvérisation placée en position "basse" sur l'appareil (débit de l'empois 10 g/min, pression d'air de pulvérisation : 105 Pa). Un granulé sec à 3,5 % d'humidité est ainsi obtenu en une seule étape.

La répartition granulométrique est la suivante :

12 % < 90 $\mu$m
23 % entre 90 et 125 $\mu$m
35 % entre 125 et 120 $\mu$m
23 % entre 180 et 250 $\mu$m
7 % > à 250 $\mu$m

Le temps d'écoulement de ce granulé est de 13 s.

Un comprimé réalisé à partir de ce granulé a une dureté de 50 N pour une force appliquée de 2 000 daN.

Une dureté de 90 N a été mesurée avec un grain obtenu dans les mêmes conditions mais présentant une humidité résiduelle de 12 %.

TABLEAU 1

CARACTERISTIQUES DES GRAINS D'AMIDON SELON L'INVENTION

COMPARATIVEMENT A UN AMIDON COMMERCIAL

| AMIDON (1500) | EXEMPLE 1 | EXEMPLE 2 | EXEMPLE 3 | COMMERCIAL (Starch 1500) |
|---|---|---|---|---|
| Granulométrie % | | | | |
| Ø > 315 μm | 25 | 16 | 10 | 0 - 5 |
| 200 < Ø < 315 μm | 25 | 16 | 30 | |
| 125 < Ø < 200 μm | 23 | 25 | 33 | 5 - 30 |
| 80 < Ø < 125 μm | 12 | 24 | 22 | |
| Ø < 80 μm | 15 | 19 | 5 | 55 - 35 |
| Temps écoulement (s) | 3,2 | 3,5 | 3 | ∞ |
| Volume de tassement (ml) | 18 | 22 | 18 | |
| Force appliquée (daN) | 2030 | | 2194 | 2140 |
| Dureté (N) | 100 | | 107 | 88 |
| Indice de cohésion | 203 | 166 | 205 | 242 |
| Friabilité (%) | 0,2 | 1,1 | 0,4 | 1 |

TABLEAU 2

| CARACTERISTIQUES DES FORMULES REALISEES AVEC UN AMIDON COMMERCIAL ET L'AMIDON SELON L'INVENTION | | | |
|---|---|---|---|
| AMIDON | EXEMPLE 1 | EXEMPLE 3 | COMMERCIAL |
| FORMULE 1 | | | |
| Force appliquée (daN) | 2020 | 2080 | 2320 |
| Dureté (N) | 73 | 78 | 67 |
| Indice de cohésion | 277 | 260 | 346 |
| Friabilité (%) | 1,0 | 0,9 | 1,22 |
| Uniformité de poids des comprimés (%) | 1,8 | 1,7 | 1,5 |
| Temps de délitement (min) | 1 | < 1 | 6,5 |
| FORMULE 2 | | | |
| Force appliquée (daN) | 1686 | 2140 | 1741 |
| Dureté (N) | 74 | 91 | 74 |
| Indice de cohésion | 227 | 235 | 236 |
| Friabilité (%) | 0,5 | 0,6 | 0,8 |
| Uniformité de poids | 1,3 | 1,3 | 2,9 |
| Temps de délitement (min) | 3 | 3 | 13 |

**Revendications**

1. Procédé de préparation de granulés d'amidon, caractérisé en ce qu'on réalise une granulation par pulvérisation, sur 99 à 80 % en poids de grains d'amidon natif, de 1 à 20 % d'amidon soluble sous forme d'un empois, obtenu dans des conditions douces le granulé obtenu ayant après séchage et, éventuellement, broyage et tamisage, une taille moyenne de particules comprise entre 100 et 500 $\mu$m.

2. Procédé selon la revendication 1, caractérisé en ce que l'on pulvérise de 2 à 6 % d'amidon soluble sous forme d'un empois sur 98 à 94 % de grains d'amidon natif.

3. Procédé selon l'une des revendications 1 ou 2, caractérisé en ce que l'empois d'amidon est obtenu par chauffage d'un amidon natif dispersé dans de l'eau à une température d'environ 85° C pendant une durée de l'ordre de 15 à 45 min.

4. Procédé selon la revendication 3, caractérisé en ce que ledit empois d'amidon a une viscosité inférieure à 1 500 mPa.s environ à 85° C pour une solution à 5 % en poids.

5. Procédé selon l'une des revendications 1 à 3, caractérisé en ce que la concentration en amidon dans l'empois est comprise entre 2 et 10 % en poids, de préférence 5 à 8 %.

6. Procédé selon l'une des revendications 1 à 4, caractérisé en ce que l'empois d'amidon obtenu est utilisé à une température supérieure à 60° C.

7. Procédé selon l'une des revendications 1 à 6, caractérisé én ce que l'empois d'amidon utilisé est obtenu à partir du même amidon natif que celui sur lequel il est pulvérisé.

8. Nouveaux granulés d'amidon, caractérisés en ce qu'ils sont obtenus par collage, de préférence ponctuel, de grains d'amidon natif entre eux, au moyen d'empois d'amidon et qu'ils présentent une granulométrie moyenne comprise entre environ 100 et 500 $\mu$m, qu'ils donnent par compression sous 20 daN des comprimés ayant une dureté d'au moins 50 N environ et se désintègrent dans l'eau en moins de 5 min.

9. Comprimés, caractérisés en ce qu'ils contiennent comme excipient des nouveaux granulés d'amidon selon la revendication 8.